# EUROPEAN PATENT SPECIFICATION

(11) **EP 1 768 581 B1**
(45) Date of publication and mention of the grant of the patent: **21.04.2010**
(21) Application number: 05750247.8
(22) Date of filing: 20.06.2005
(51) Int. Cl.: A61B 17/50

(54) **APPARATUS FOR REMOVING TICKS**
GERÄT ZUR ENTFERNUNG VON ZECKEN
APPAREIL D'ELIMINATION DES TIQUES

(30) Priority: 21.06.2004 AU 2004100487; 21.06.2004 AU 2004903331
(43) Date of publication of application: 04.04.2007
(73) Proprietor: Veterinary Companies of Australia Pty Ltd, Kings Park, NSW 2148 (AU)
(72) Inventor: BLOOM, Lionel, Kings Park, NSW 2148 (AU)
(74) Representative: Wilson Gunn
(86) International application number: PCT/AU2005/000885
(87) International publication number: WO 2005/122920

(56) References cited:
- WO-A-96/38095
- WO-A1-96/38095
- WO-A1-2004/054457
- AU-B4- 2004 100 487
- DE-U1- 20 005 472
- DE-U1- 20 005 472
- DE-U1- 20 317 698
- DE-U1- 29 722 310
- DE-U1- 29 722 310
- US-A- 5 447 511
- US-A- 5 447 511
- US-A- 5 607 434
- US-A- 5 607 434
- US-A- 5 843 094
- US-A- 5 843 094
- US-A- 6 102 919
- US-A- 6 102 919

## Description

### FIELD OF THE INVENTION

This invention relates to an improved apparatus for removing ticks and the like from the skin of animals and humans.

### BACKGROUND OF THE INVENTION

Ticks are very hazardous to the health of animals and humans. They infect the victim by penetrating the epidermis with their rostrum and thus remain adhered to the skin until forcibly removed. Ticks often carry bacteria which may be passed to the animal or human whilst the tick is engorging itself on the carriers blood. In humans this can lead to paralysis, allergic reactions and in some cases Lyme Disease (a disorder having severe flu like symptoms). In animals, tick infestations can lead to paralysis and in severe cases death.

Traditionally ticks are removed using tweezers, burning them off and/or application of acaricide preparation. Tweezers have the advantage in that they can be used for various sized ticks. However they can result in the body of the tick being squashed and host being covered in blood and saliva.

Australian patent AU 6 227 896, describes an implement having an elongated handle with a perpendicular forked region. The forked region is fitted around the head and thorax of the tick, the handle of the implement rotated until the tick is removed from the skin. A number of implements with differing sized forked regions are required to be utilised for the removal of ticks of varying sizes.

### OBJECT OF THE INVENTION

It is an object of the invention to overcome or alleviate one or more of the above disadvantages or to provide the consumer with a useful or commercial choice.

### SUMMARY OF THE INVENTION

In one form, although not necessarily the broadest or only form, the invention resides in an apparatus, for removing ticks from animals and humans, comprising:
an elongated grip having a longitudinal axis;
a first bifurcated tip located at one end of the elongated grip; and
a second bifurcated tip located at a second end of the elongated grip,
wherein in the first and second bifurcated tips extending substantially perpendicular to the longitudinal axis of the elongated grip and the second bifurcated tip is smaller than the first bifurcated tip.

The elongated grip is preferably cylindrical in nature. The surface of the elongated grip may be contoured or textured to facilitate gripping in the users hand. The first and second bifurcated tips may be rectilinear or curved towards the elongated grip. The first and second bifurcated tips may extend substantially perpendicular from the longitudinal axis of the elongated grip in the same or opposing directions. The bifurcated tips are preferably chamfered at their extremity. The tips are bifurcated to form prongs. The space between the prongs preferably forms an elongated V shape adapted to releasably engage a tick for removal. Preferably, the second bifurcated tip is smaller than the first bifurcated tip.

### GRIEF DESCRIPTION OF THE DRAWINGS

Embodiments of the invention will be described, by way of example only, with reference to the accompanying drawings in which:

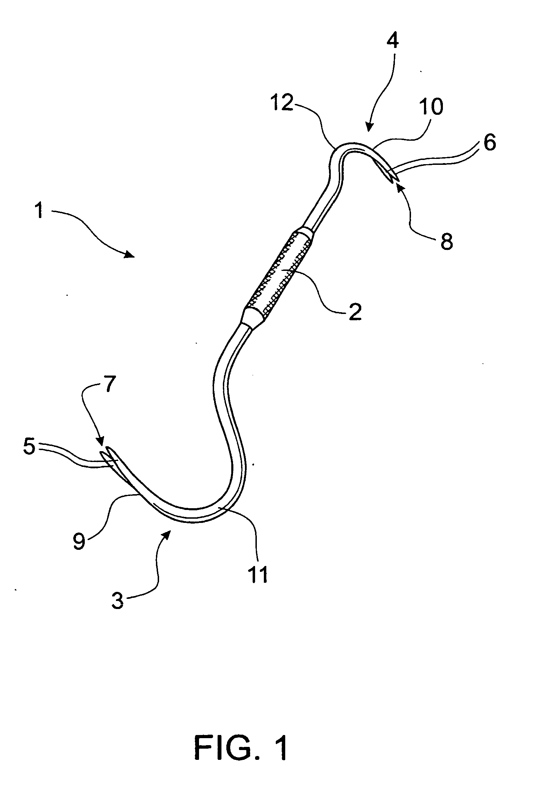

FIG. 1 is a schematic representation of an embodiment of the apparatus of the invention.

### DETAILED DESCRIPTION

FIG. 1 shows an apparatus or tick remover 1 having an elongated grip 2. In this embodiment the elongated grip 2 is cylindrical in nature and has a fine stipple finish. It will be appreciated that the elongated grip 2 may have a range of surface textures to facilitate gripping, including smooth, rippled, striated and the like. Furthermore, the elongated grip 2 may be contoured; cylindrical; and/or be square, hexagonal or octagonal in cross-section to facilitate gripping by the user's hand.

At one end of the elongated grip 2 extends a first bifurcated tip 3. At a second end of the elongated grip 2 is located a second bifurcated tip 4. The bifurcated tips 3 and 4 have two prongs each, 5 and 6 respectively. The leading edge of the prongs 5 and 6 is chamfered. The prongs 5 and 6 define an elongated V-shaped space 7 and 8, respectively. The bifurcated tips 3 and 4 have an outer surface 9 and 10, respectively, which is substantially flat to allow for sufficient contact with the skin of the infected animal or human.

In this embodiment the first and second bifurcated tips 3 and 4 extend from the elongated grip 2 in a curved manner, such as a goose-neck shape, shown as 11 and 12, respectively in FIG 1, and extend in opposite directions from the longitudinal axis of the elongated grip 2. It will be appreciated that the curved sections 11 and 12 may be replaced with linear or angled sections. Furthermore, the curved sections 11 and 12 may extend in the same direction from the longitudinal axis of the elongated grip 2.

The first bifurcated tip 3 in this embodiment is shown as being larger in overall size and depth of the elongated V-shaped space 7 than the second bifurcated tip 4 and its related elongated V-shaped space 8, to allow for the tick remover 1 to be readily used in the removal of different sized ticks. It will be appreciated that the first and second bifurcated tips, 3 and 4, may be the same size and have the same or different depth of their respective elongated V-shaped space, 7 and 8.

In use the tick remover 1 is held in the hand of the user by the elongated grip 2 and either the first or second bifurcated tip (3 or 4) nears the tick located in the skin of an animal or human with the pair of prongs (5 or 6) of the first or second bifurcated tip (3 or 4) directed towards the tick. The prongs (5 or 6) of the tip are slid underneath the tick to result in the tick being located firmly between the prongs (5 or 6). The user then applies a slight pulling force away from the skin prior to rotating or twisting the apparatus between the thumb and fore index finger until the tick loosens. The ticks generally loosen or are removed with the second or third twist of the apparatus. If preferred the user may then apply antiseptic.

Ticks may vary significantly in size through their stages of development from larvae, pupa or adults and with the variety of tick species. Furthermore a tick may become significantly engorged with blood if it has been located on the skin for an extended period of time. The tick removal apparatus of the invention provides an effective way of removing ticks from the skin of animals or humans regardless of the size of the tick to be removed without the inconvenience of having multiple sized tools, which may be easily lost or make removing many ticks from an infected animal a very time consuming exercise, or risking squashing the ticks during removal.

## Claims

1. An apparatus (1), for removing ticks from animals and humans, comprising:
an elongated grip (2) having a longitudinal axis and
a first bifurcated tip (3) located at one end of the elongated grip (2); **characterized in that** it further comprises
a second bifurcated tip (4) located at a second end of the elongated grip (2),
wherein in the first and second bifurcated tips extending substantially perpendicular to the longitudinal axis of the elongated grip (2) and the second bifurcated tip (4) is smaller than the first bifurcated tip (3).

2. The apparatus of claim 1, wherein the first (3) and second (4) bifurcated tips are curved towards the elongated grip (2).

3. The apparatus of claim 1, wherein the first (3) and second (4) bifurcated tips are rectilinear to the elongated grip (2).

4. The apparatus of any one of the preceding claims, wherein the first (3) and second (4) bifurcated tips extend substantially perpendicular from the longitudinal axis of the elongated grip (2) in opposing directions.

5. The apparatus of any one of the preceding claims, wherein the first (3) and second (4) bifurcated tips extend substantially perpendicular from the longitudinal axis of the elongated grip (2) in the same direction.

6. The apparatus of any one of the preceding claims, wherein the first (3) and second (4) bifurcated tips are bifurcated to form prongs (5, 6), which form an elongated V-shaped space between the prongs (5, 6) adapted to releasably engage a tick for removal.

7. The apparatus of any one of the preceding claims, wherein the bifurcated tips (3), (4) are chamfered at their extremities.

## Patentansprüche

1. Vorrichtung (1) zum Entfernen von Zecken von Tieren und Menschen, aufweisend:
einen länglichen Griff (2) mit einer Längsachse und
eine erste gegabelte Spitze (3), die sich an einem Ende des länglichen Griffs (2) befindet;
**dadurch gekennzeichnet,**
**dass** sie weiterhin eine zweite gegabelte Spitze (4) aufweist, die sich an einem zweiten Ende des länglichen Griffs (2) befindet,
wobei sich die erste und die zweite gegabelte Spitze im Wesentlichen senkrecht zur Längsachse des länglichen Griffs (2) erstrecken und die zweite gegabelte Spitze (4) kleiner als die erste gegabelte Spitze (3) ist.

2. Vorrichtung nach Anspruch 1,
wobei die erste (3) und zweite (4) gegabelte Spitze zum länglichen Griff (2) hin gebogen sind.

3. Vorrichtung nach Anspruch 1,
wobei die erste (3) und zweite (4) gegabelte Spitze geradlinig zu dem länglichen Griff (2) sind.

4. Vorrichtung nach einem der vorangegangenen Ansprüche,
wobei sich die erste (3) und zweite (4) gegabelte Spitze im Wesentlichen senkrecht von der Längsachse des länglichen Griffs (2) in entgegengesetzte Richtungen erstrecken.

5. Vorrichtung nach einem der vorangegangenen Ansprüche,
wobei sich die erste (3) und zweite (4) gegabelte Spitze im Wesentlichen senkrecht von der Längsachse des länglichen Griffs (2) in dieselbe Richtung erstrecken.

6. Vorrichtung nach einem der vorangegangenen Ansprüche,
wobei die erste (3) und zweite (4) gegabelte Spitze derart gegabelt sind, dass sie Zinken (5, 6) bilden, welche einen länglichen V-förmigen Raum zwischen den Zinken (5, 6) bilden, der dazu geeignet ist, eine Zecke zum Entfernen lösbar zu greifen.

7. Vorrichtung nach einem der vorangegangenen Ansprüche,
wobei die gegabelten Spitzen (3, 4) an ihren Enden angeschrägt sind.

## Revendications

1. Appareil (1) pour éliminer les tiques sur les animaux et les êtres humains, comprenant :
une poignée allongée (2) ayant un axe longitudinal et
une première pointe fourchue (3) située à une extrémité de la poignée allongée (2) ; **caractérisé en ce qu'**il comprend en outré :
une seconde pointe fourchue (4) située à une seconde extrémité de la poignée allongée (2),
dans lequel les première et seconde pointes fourchues s'étendent de manière sensiblement perpendiculaire à l'axe longitudinal de la poignée allongée (2) et la seconde pointe fourchue (4) est plus petite que la première pointe fourchue (3).

2. Appareil selon la revendication 1, dans lequel les première (3) et seconde (4) pointes fourchues sont incurvées vers la poignée allongée (2).

3. Appareil selon la revendication 1, dans lequel les première (3) et seconde (4) pointes fourchues sont rectilignes par rapport à la poignée allongée (2).

4. Appareil selon l'une quelconque des revendications précédentes, dans lequel les première (3) et seconde (4) pointes fourchues s'étendent de manière sensiblement perpendiculaire à l'axe longitudinal de la poignée allongée (2) dans des sens opposés.

5. Appareil selon l'une quelconque des revendications précédentes, dans lequel les première (3) et seconde (4) pointes fourchues s'étendent de manière sensiblement perpendiculaire à l'axe longitudinal de la poignée allongée (2) dans le même sens.

6. Appareil selon l'une quelconque des revendications précédentes, dans lequel les première (3) et seconde (4) pointes fourchues sont fourchues pour former des dents (5, 6) qui forment un espace allongé en forme de V entre les dents (5, 6) adapté pour s'engager de manière libérable sur une tique à éliminer.

7. Appareil selon l'une quelconque des revendications précédentes, dans lequel les pointes fourchues (3, 4) sont biseautées à leurs extrémités.
